# EUROPEAN PATENT APPLICATION

(11) **EP 3 485 817 A1**
(43) Date of publication of application: **22.05.2019**
(21) Application number: 18204604.5
(22) Date of filing: 06.11.2018
(51) Int. Cl.: A61B 8/00, F16F 15/14, H02K 5/24, G01S 15/89

(54) **VIBRATION CANCELING MOTOR ASSEMBLY AND ULTRASOUND PROBE INCLUDING THE SAME**

(30) Priority: 16.11.2017 KR 20170153325
(71) Applicant: Samsung Medison Co., Ltd., Hongcheon-gun, Gangwon-do, 25108 (KR)
(72) Inventor: Won, Jong Sik, 13530 Gyeonggi-do (KR); Kwon, Gi Han, 13530 Gyeonggi-do (KR); Yoo, Jae Won, 13530 Gyeonggi-do (KR); Lee, Jae Wook, 13530 Gyeonggi-do (KR); Cho, Jeong, 13530 Gyeonggi-do (KR)
(74) Representative: Hylarides, Paul Jacques

(57) **Abstract**

A vibration canceling motor assembly and an ultrasound probe are provided, including a driving force transmission member rotating in a state of being fixed to a shaft and transmitting a driving force to outside, a mass body arranged on at least a portion of an outer circumferential surface of the driving force transmission member, and an adhesion member configured to couple the mass body to the driving force transmission member. Accordingly, a vibration is efficiently attenuated.

## Description

### BACKGROUND

### 1. Field

The disclosure relates to a vibration canceling motor assembly and an ultrasound probe including the vibration canceling motor assembly.

### 2. Description of the Related Art

Ultrasound diagnosis apparatuses transmit an ultrasound signal generated by a transducer of a probe to an object and receives information regarding a signal reflected from the object, thereby obtaining at least one image of a part (e.g., a soft tissue or a blood stream) inside the object.

Ultrasound probes include a power unit that provides power that is required to drive a transducer. In general, the power unit includes a motor that rotates a shaft. Because an ultrasound probe needs to scan an object with ultrasounds in any direction in a one-dimensional (ID) or two-dimensional (2D) manner, a rotation direction of a motor that drives the ultrasound probe is also frequently changed. Thus, the motor is greatly vibrated, and accordingly a user may feel fatigue when using the ultrasound probe for a long time.

### SUMMARY

Provided are a vibration canceling motor assembly and an ultrasound probe including the vibration canceling motor assembly.

Additional aspects will be set forth in part in the description which follows and, in part, will be apparent from the description, or may be learned by practice of the presented embodiments.

In accordance with an aspect of the disclosure, a vibration canceling motor assembly includes a motor; a shaft that is rotated about a rotation axis of the shaft by the motor; a driving force transmission member including a support area coupled to the shaft to be fixed to the shaft and a drive transmission area that transmits a driving force to outside; a mass body arranged in at least a portion of the support area; and an adhesion member interposed between the mass body and the driving force transmission member.

The driving force transmission member may have a hollow shape such that the shaft is inserted into and fixed to the driving force transmission member.

The shaft may include a shaft groove formed on at least a portion of an outer circumferential surface of the shaft, and the driving force transmission member may include a protrusion inserted into the shaft groove to couple the shaft to the driving force transmission member.

The shaft groove may be provided on a leading end of the shaft.

The vibration canceling motor assembly may further include a coupling member configured to couple the adhesion member to the mass body.

The coupling member may be further configured to couple the adhesion member to the driving force transmission member.

The coupling member may be arranged to penetrate the adhesion member and the support area of the driving force transmission member.

The mass body may include an insertion portion extending in a direction perpendicular to the rotation axis of the shaft and allowing the coupling member to be inserted into the insertion portion.

The vibration canceling motor assembly may further include an insertion portion mass body arranged in the insertion portion.

A density distribution of the mass body may be non-uniform in a radial direction perpendicular to the rotation axis of the shaft.

A density of the mass body may increase in the radial direction perpendicular to the rotation axis of the shaft.

The mass body may include a plurality of areas having different densities.

The mass body may include a first mass body and a second mass body sequentially arranged in the radial direction perpendicular to the rotation axis of the shaft, and a density of the second mass body is higher than a density of the first mass body.

The driving force transmission member may be a bending gear or a pulley gear.

The adhesion member may include an elastic material having an elasticity.

The mass body may include a metal material.

A natural frequency of the mass body may be substantially the same as a natural frequency of the motor.

A natural frequency of the adhesion member may be substantially the same as a natural frequency of the motor.

In accordance with another aspect of the disclosure, an ultrasound probe includes the above-described vibration canceling motor assembly.

The ultrasound probe may further include an ultrasound module including a transducer or a piezoelectric element and configured to generate ultrasound waves; and a drive transmitter configured to transmit a drive from the vibration canceling motor assembly to the ultrasound module.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a block diagram illustrating an ultrasound diagnosis apparatus according to an exemplary embodiment;
FIG. 2 is a schematic view illustrating a structure of an ultrasound probe according to an embodiment;
FIG. 3 is a schematic cross-section of a vibration canceling motor assembly according to an embodiment;
FIG. 4 is a schematic cross-section of a vibration canceling motor assembly according to another embodiment;
FIG. 5 is a schematic cross-section of a vibration canceling motor assembly according to another embodiment;
FIG. 6 illustrates a cross-section of a leading end of a shaft of the vibration canceling motor assembly of FIG. 5 and a cross-section of a leading end of a driving force transmission member of the vibration canceling motor assembly 3 of FIG. 5;
FIG. 7 is a schematic cross-section of a vibration canceling motor assembly according to another embodiment;
FIG. 8 is a schematic cross-section of a vibration canceling motor assembly according to another embodiment;
FIG. 9 is a schematic cross-section of a vibration canceling motor assembly according to another embodiment; and
FIG. 10 is a schematic cross-section of a vibration canceling motor assembly according to another embodiment.

### DETAILED DESCRIPTION

Certain exemplary embodiments are described in greater detail below with reference to the accompanying drawings.

In the following description, the same drawing reference numerals are used for the same elements even in different drawings. The matters defined in the description, such as detailed construction and elements, are provided to assist in a comprehensive understanding of exemplary embodiments. Thus, it is apparent that exemplary embodiments can be carried out without those specifically defined matters. Also, well-known functions or constructions are not described in detail since they would obscure exemplary embodiments with unnecessary detail.

Terms such as "part" and "portion" used herein denote those that may be embodied by software or hardware. According to exemplary embodiments, a plurality of parts or portions may be embodied by a single unit or element, or a single part or portion may include a plurality of elements.

In exemplary embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ or a tissue, or a phantom.

Throughout the specification, an ultrasound image refers to an image of an object processed based on ultrasound signals transmitted to the object and reflected therefrom.

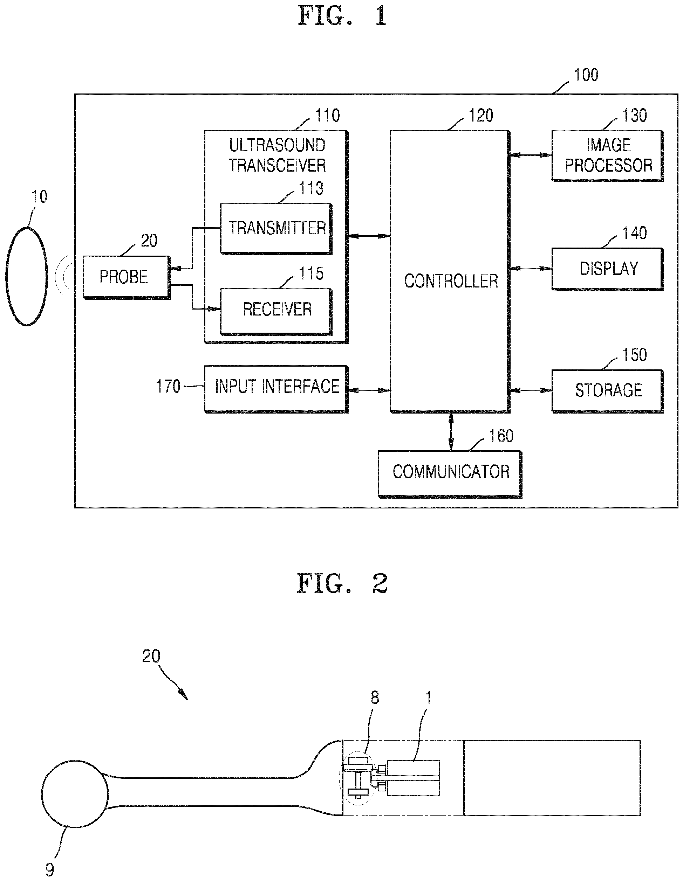

FIG. 1 is a block diagram illustrating a configuration of an ultrasound diagnosis apparatus 100, i.e., a diagnostic apparatus, according to an exemplary embodiment.

Referring to FIG. 1, the ultrasound diagnosis apparatus 100 may include a probe 20, an ultrasound transceiver 110, a controller 120, an image processor 130, one or more displays 140, a storage 150, e.g., a memory, a communicator 160, i.e., a communication device or an interface, and an input interface 170.

The ultrasound diagnosis apparatus 100 may be of a cart-type or a portable-type ultrasound diagnosis apparatus, that is portable, moveable, mobile, or hand-held. Examples of the portable-type ultrasound diagnosis apparatus 100 may include a smart phone, a laptop computer, a personal digital assistant (PDA), and a tablet personal computer (PC), each of which may include a probe and a software application, but embodiments are not limited thereto.

The probe 20 may include a plurality of transducers. The plurality of transducers may transmit ultrasound signals to an object 10 in response to transmitting signals received by the probe 20, from a transmitter 113. The plurality of transducers may receive ultrasound signals reflected from the object 10 to generate reception signals. In addition, the probe 20 and the ultrasound diagnosis apparatus 100 may be formed in one body (e.g., disposed in a single housing), or the probe 20 and the ultrasound diagnosis apparatus 100 may be formed separately (e.g., disposed separately in separate housings) but linked wirelessly or via wires. In addition, the ultrasound diagnosis apparatus 100 may include one or more probes 20 according to embodiments.

The controller 120 may control the transmitter 113 for the transmitter 113 to generate transmitting signals to be applied to each of the plurality of transducers based on a position and a focal point of the plurality of transducers included in the probe 20.

The controller 120 may control the ultrasound receiver 115 to generate ultrasound data by converting reception signals received from the probe 20 from analogue to digital signals and summing the reception signals converted into digital form, based on a position and a focal point of the plurality of transducers.

The image processor 130 may generate an ultrasound image by using ultrasound data generated from the ultrasound receiver 115.

The display 140 may display a generated ultrasound image and various pieces of information processed by the ultrasound diagnosis apparatus 100. The ultrasound diagnosis apparatus 100 may include two or more displays 140 according to the present exemplary embodiment. The display 140 may include a touch screen in combination with a touch panel.

The controller 120 may control the operations of the ultrasound diagnosis apparatus 100 and flow of signals between the internal elements of the ultrasound diagnosis apparatus 100. The controller 120 may include a memory for storing a program or data to perform functions of the ultrasound diagnosis apparatus 100 and a processor and/or a microprocessor (not shown) for processing the program or data. For example, the controller 120 may control the operation of the ultrasound diagnosis apparatus 100 by receiving a control signal from the input interface 170 or an external apparatus.

The ultrasound diagnosis apparatus 100 may include the communicator 160 and may be connected to external apparatuses, for example, servers, medical apparatuses, and portable devices such as smart phones, tablet personal computers (PCs), wearable devices, etc., via the communicator 160.

The communicator 160 may include at least one element capable of communicating with the external apparatuses. For example, the communicator 160 may include at least one among a short-range communication module, a wired communication module, and a wireless communication module.

The communicator 160 may receive a control signal and data from an external apparatus and transmit the received control signal to the controller 120 so that the controller 120 may control the ultrasound diagnosis apparatus 100 in response to the received control signal.

The controller 120 may transmit a control signal to the external apparatus via the communicator 160 so that the external apparatus may be controlled in response to the control signal of the controller 120.

For example, the external apparatus connected to the ultrasound diagnosis apparatus 100 may process the data of the external apparatus in response to the control signal of the controller 120 received via the communicator 160.

A program for controlling the ultrasound diagnosis apparatus 100 may be installed in the external apparatus. The program may include command languages to perform part of operation of the controller 120 or the entire operation of the controller 120.

The program may be pre-installed in the external apparatus or may be installed by a user of the external apparatus by downloading the program from a server that provides applications. The server that provides applications may include a recording medium where the program is stored.

The storage 150 may store various data or programs for driving and controlling the ultrasound diagnosis apparatus 100, input and/or output ultrasound data, ultrasound images, applications, etc.

The input interface 170 may receive a user's input to control the ultrasound diagnosis apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto.

FIG. 2 is a schematic view illustrating a structure of the probe 20 , according to an embodiment. Referring to FIG. 2, the probe 20 may include a vibration canceling motor assembly 1, a drive transmitter 8, and an ultrasound module 9. The drive transmitter 8 may include a bending gear, a pulley gear, a guide member, a rotation member, and a wire member for transmitting a drive to the ultrasound module 9, but the present disclosure is not limited to the particular embodiment. The ultrasound module 9 may include a component that generates ultrasound waves, based on a driving force. For example, the ultrasound module 9 may include a transducer or a piezoelectric element. For example, the ultrasound module 9 may include a one-dimensional (ID) or two-dimensional (2D) piezoelectric element array or transducer array. The vibration canceling motor assembly 1 may attenuate a vibration and also transmit a generated drive to the ultrasound module 9 via the drive transmitter 8. According to the present embodiment, the probe 20 may reduce displeasures of a user and an object because a vibration is reduced. A detailed structure of the vibration canceling motor assembly 1 will now be described.

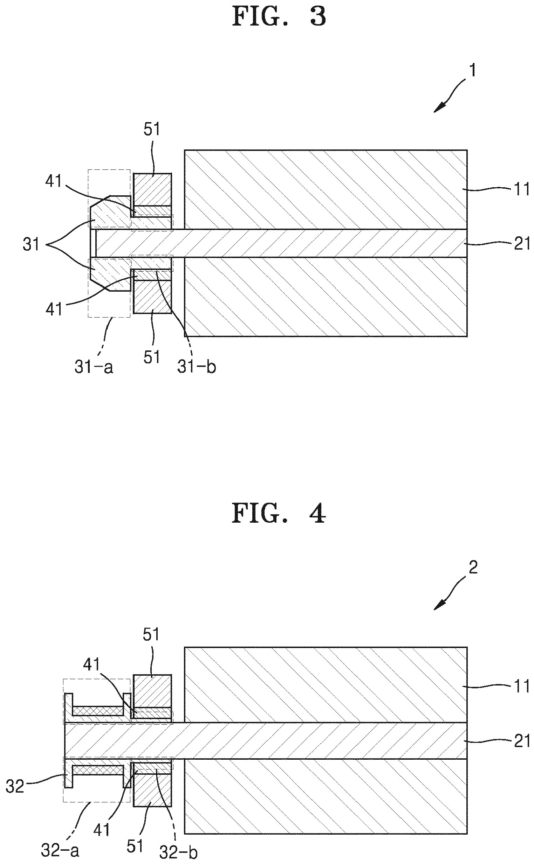

FIG. 3 is a schematic cross-section of the vibration canceling motor assembly 1, according to an embodiment. Referring to FIG. 3, the vibration canceling motor assembly 1 includes a motor 11, a shaft 21, a driving force transmission member 31, an adhesion member 41, and a mass body 51.

The motor 11 may rotate the shaft 21, about a rotation axis of the shaft 21. The motor 11 may change a rotation direction of the shaft 21, under the control of a controller (not shown). The shaft 21 may be coupled with the driving force transmission member 31 and thus may be fixed thereto.

The driving force transmission member 31 may transmit a driving force to outside of the motor 11 by being engaged with another driving force transmission member (not shown) or being connected to a belt (not shown). For example, the driving force transmission member 31 may transmit a driving force generated by the motor 11 to the ultrasound module 9 of FIG. 2. The driving force transmission member 31 may be formed of metal or plastic. The driving force transmission member 31 may be, for example, a bending gear.

The driving force transmission member 31 may include a support area 31-b coupled with the shaft 21 to be fixed thereto, and a drive transmission area 31-a that transmits a driving force to outside. The drive transmission area 31-a means an area that is engaged with, contact, or is coupled to an external member (for example, the drive transmitter 8 of FIG. 2) to transmit a driving force to the outside. For example, the drive transmission area 31-a according to the present embodiment may mean the whole area that contacts an external bending gear (not shown). The support area 31-b means an area that provides a coupling force such that the driving force transmission member 31 may be fixed to the shaft 21. For example, according to the present embodiment, the support area 31-b may be defined as an area adjacent to the drive transmission area 31-a.

The driving force transmission member 31 may be formed to have a hollow shape such that the shaft 21 may be inserted into and fixed to the hollow driving force transmission member 31. For example, respective inner circumferential surfaces of the drive transmission area 31-a and the support area 31-b may be engaged with an outer circumferential surface of the shaft 21 via a frictional force. For example, not only the support area 31-b but also the drive transmission area 31-a may provide a coupling force via by being fixed to the shaft 21.

The drive transmission area 31-a and the support area 31-b may be arranged side by side in a lengthwise direction of the shaft 21. For example, referring to FIG. 3, the support area 31-b and the transmission area 31-a may be sequentially arranged based on the motor 11. However, the present disclosure is not limited thereto, and the transmission area 31-a and the support area 31-b may be sequentially arranged based on the motor 11.

A radial length of the support area 31-b in a vertical direction of the rotation axis of the shaft 21 may be less than that of the drive transmission area 31-a in the vertical direction of the rotation axis. For example, when the radial length of the support area 31-b in the vertical direction of the rotation axis of the shaft 21 is small, an area where the mass body 51 and the adhesion member 41 are to be arranged may be sufficiently secured. For example, the radial length of the support area 31-b in the vertical direction of the rotation axis of the shaft 21 may be designed to be equal to or greater than a certain length to have sufficient rigidity for coupling the driving force transmission member 31 to the shaft 21.

A length of the support area 31-b in a lengthwise direction of the rotation axis of the shaft 21 may be determined within a length obtained by subtracting the drive transmission area 31-a from a length of a portion of the shaft 21 that protrudes from the motor 11. For example, the support area 31-b may have a maximum length within a range in which the support area 31-b does not contact the motor 11. However, the present disclosure is not limited thereto.

The mass body 51 may be formed on an outer circumferential surface of the driving force transmission member 31 in a circumferential direction. For example, the mass body 51 may be matched with at least a portion of the support area 31-b. The mass body 51 may attenuate a vibration formed in the motor 11. The mass body 51 may have a natural frequency for causing a resonance with the motor 11. For example, the natural frequency of the mass body 51 may be substantially the same as that of the motor 11. The natural frequency of the mass body 51 may be determined according to a design element, such as a material, rigidity, hardness, mass, shape, volume, and density of the mass body 51.

The mass body 51 may have an appropriate shape to absorb the vibration of the motor 11. For example, the mass body 51 may have a shape that is symmetrical about the rotation axis of the shaft 21. For example, the mass body 51 may have a hollow disk shape, but the present disclosure is not limited thereto. A vibration attenuation performance of the mass body 51 may be favorable as a center of mass of the mass body 51 is located farther from the rotation axis of the shaft 21. The vibration attenuation performance of the mass body 51 may be favorable as a rotational inertia of the mass body 51 is high.

The mass body 51 may be formed of, for example, metal. The mass body 51 may be formed of palladium (Pd), platinum (Pt), ruthenium (Ru), gold (Au), silver (Ag), molybdenum (Mo), magnesium (Mg), aluminum (Al), tungsten (W), titanium (Ti), iridium (Ir), nickel (Ni), chromium (Cr), neodymium (Nd), or copper (Cu).

The adhesion member 41 may adhere the mass body 51 and the driving force transmission member 31 together. The adhesion member 41 may have a natural frequency for attenuating the vibration formed in the motor 11. The natural frequency of the adhesion member 41 may be substantially the same as that of the motor 11. The natural frequency of the adhesion member 41 may be determined according to a design element, such as a material, rigidity, hardness, mass, shape, volume, and density of the adhesion member 41.

The adhesion member 41 may be formed to fill an interval between the mass body 51 and the driving force transmission member 31. A bonding strength provided by the adhesion member 41 may be controlled according to an area of an interval between the mass body 51 and the driving force transmission member 31, a volume of the interval, and a material used to form the adhesion member 41. The shape of the adhesion member 41 may be determined according to a shape of the interval between the mass body 51 and the driving force transmission member 31. For example, the shape of the adhesion member 41 may be a hollow disk shape.

The adhesion member 41 may include, for example, an elastic material including certain elasticity. For example, the adhesion member 41 may be formed of a rubber material. For example, the adhesion member 41 may be formed by flowing liquefied rubber into the interval between the mass body 51 and the driving force transmission member 31 and hardening the liquefied rubber. The adhesion member 41 may provide a bonding strength for fixing the mass body 51 to the driving force transmission member 31, and an elastic force for reducing a friction, at the same time.

The vibration canceling motor assembly 1 according to the present embodiment may effectively attenuate the vibration of the motor 11 by providing the adhesion member 41 and the mass body 51 on the outer circumferential surface of the driving force transmission member 31. This vibration canceling motor assembly 1 may provide a good effect, compared with a comparative example in which a shaft extends to a rear of a motor and an elasticity attenuation structure is formed on the shaft. For example, the vibration canceling motor assembly 1 according to the present embodiment may have a relatively small volume and provide good vibration attenuation efficiency, compared with an assembly according to the above-described comparative example. This is because, in the vibration canceling motor assembly 1 according to the present embodiment, the mass body 51 and the adhesion member 41 are provided directly on the driving force transmission member 31 instead of the shaft 21. It is assumed that an adhesion member 41 and a mass body 51 having the same mass and the same volume are provided for vibration attenuation. In the vibration canceling motor assembly 1 according to the present embodiment, the adhesion member 41 and the mass body 51 are located far from the rotation axis of the shaft 21, and thus the adhesion member 41 and the mass body 51 have large rotational inertias. Because the adhesion member 41 and the mass body 51 are provided directly on the driving force transmission member 31, an additional space (i.e., a rear space of the motor 11) is not needed, and thus an ultrasound probe may be favorably integrated.

FIG. 4 is a schematic cross-section of a vibration canceling motor assembly 2 according to another embodiment. Referring to FIG. 3, the vibration canceling motor assembly 2 includes a motor 11, a shaft 21, a driving force transmission member 32, an adhesion member 41, and a mass body 51. Components of the vibration canceling motor assembly 2 except for the driving force transmission member 32 are the same as those of the vibration canceling motor assembly of FIG. 2, and thus a redundant description thereof will be omitted.

The driving force transmission member 32 may be a pulley gear including a drive transmission area 32-a and a support area 32-b. The drive transmission area 32-a may be an area that is connected to a belt (not shown) and transmits a driving force to the outside. The support area 32-b means an area that provides a coupling force such that the driving force transmission member 32 may be fixed to the shaft 21. For example, according to the present embodiment, the support area 32-b may be defined as an area adjacent to the drive transmission area 32-a.

The adhesion member 41 and the mass body 51 may be provided in at least a portion of the support area 32-b. The adhesion member 41 and the mass body 51 may effectively attenuate a vibration formed in rotation of the motor 11.

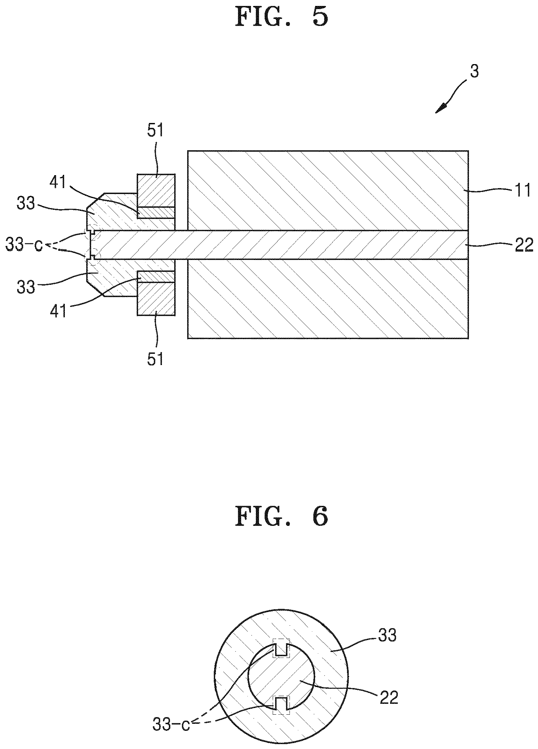

FIG. 5 is a schematic cross-section of a vibration canceling motor assembly 3 according to another embodiment. FIG. 6 illustrates a cross-section of a leading end of a shaft 22 of the vibration canceling motor assembly 3 of FIG. 5 and a cross-section of a leading end of a driving force transmission member 33 of the vibration canceling motor assembly 3 of FIG. 5.

Referring to FIGS. 5 and 6, the vibration canceling motor assembly 3 includes a motor 11, the shaft 22, the driving force transmission member 33, an adhesion member 41, and a mass body 51. Components of the vibration canceling motor assembly 3 except for the shaft 22 and the driving force transmission member 33 are the same as those of the vibration canceling motor assembly 1 of FIG.2, and thus a redundant description thereof will be omitted.

The shaft 22 may include a shaft groove in at least a portion of an outer circumferential surface of the shaft 22. The driving force transmission member 33 may include a protrusion 33-c engageable with the shaft groove, in at least a portion of an outer circumferential surface of the driving force transmission member 33. For example, the shaft groove of the shaft 22 may be formed on a leading end of the outer circumferential surface of the shaft 22. When the shaft groove of the shaft 22 may be formed on the leading end of the outer circumferential surface of the shaft 22, the driving force transmission member 33 may be coupled to the shaft 22 in a direction parallel with a rotation axis of the shaft 22, and thus the two components may be easily coupled to each other. For example, the shaft 22 may include one or more shaft grooves, and the driving force transmission member 33 may include as many protrusions 33-c as the number of shaft grooves of the shaft 22.

The shaft 22 may obtain an additional coupling force via coupling between the shaft grooves and the protrusions 33-c. A coupling structure between the shaft 22 and the driving force transmission member 33 prevents a slip phenomenon from occurring by adding the adhesion member 41 and the mass body 51 to the outer circumferential surface of the driving force transmission member 33, thereby improving a vibration attenuation effect.

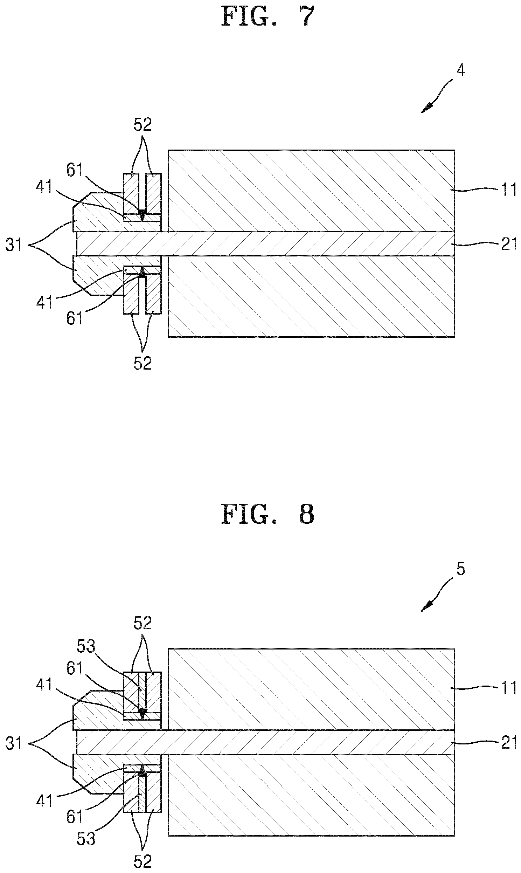

FIG. 7 is a schematic cross-section of a vibration canceling motor assembly 4 according to another embodiment. Referring to FIG. 7, the vibration canceling motor assembly 4 includes a motor 11, a shaft 21, a driving force transmission member 31, an adhesion member 41, a mass body 52, and a coupling member 61. Components of the vibration canceling motor assembly 4 except for the mass body 52 and the coupling member 61 are the same as those of the vibration canceling motor assembly 1 of FIG.2, and thus a redundant description thereof will be omitted.

The coupling member 61 may couple the mass body 52 to the adhesion member 41. When an adhesive force provided by the adhesion member 41 is insufficient to handle a centrifugal force of the mass body 52, the coupling member 61 may more strongly couple the mass body 52 to the adhesion member 41. The coupling member 61 may include a member such as a volt or a screw. The coupling member 61 may couple the mass body 52 to the adhesion member 41 and also couple the adhesion member 41 to the driving force transmission member 31. For example, when the driving force transmission member 31 is formed of a material easy to be perforated (i.e., plastic), the coupling member 61 may penetrate the mass body 52, the adhesion member 41, and the driving force transmission member 31 and provide an additional coupling force. Even when a rotational force or rotating speed of the motor 11 is high and a mass of the mass body 52 is high, the coupling member 61 may provide a sufficient coupling force such that the mass body 52, the adhesion member 41, and the driving force transmission member 31 are rotated like one body.

The mass body 52 may have an insertion portion to which the coupling member 61 is to be coupled. The insertion portion may be formed in a direction perpendicular to the rotation axis of the shaft 21 such that a vibration attenuation function of the mass body 52 is not reduced.

FIG. 8 is a schematic cross-section of a vibration canceling motor assembly 5 according to another embodiment. Referring to FIG. 8, the vibration canceling motor assembly 5 includes a motor 11, a shaft 21, a driving force transmission member 31, an adhesion member 41, a mass body 52, an insertion portion mass body 53, and a coupling member 61. Components of the vibration canceling motor assembly 5 except for the insertion portion mass body 53 are the same as those of the vibration canceling motor assembly 1 of FIG. 2, and thus a redundant description thereof will be omitted.

The insertion portion mass body 53 may fill an insertion portion of the mass body 52 formed to allow the coupling member 61 to be inserted thereinto. A vibration attenuation effect of the mass body 52 may increase with an increase in the mass of the mass body 52, without a change in a volume of the mass body 52. Accordingly, the insertion portion of the mass body 52 may be filled with the insertion portion mass body 53, and thus the vibration attenuation effect of the mass body 52 may be improved. The insertion portion mass body 53 and the mass body 52 may be formed of the same material, but the present disclosure is not limited thereto.

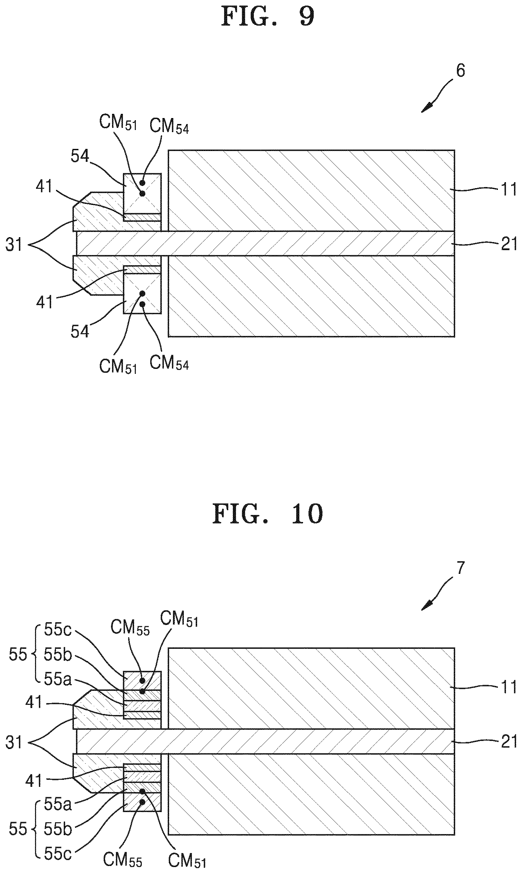

FIG. 9 is a schematic cross-section of a vibration canceling motor assembly 6 according to another embodiment. Referring to FIG. 9, the vibration canceling motor assembly 6 includes a motor 11, a shaft 21, a driving force transmission member 31, an adhesion member 41, and a mass body 54. Components of the vibration canceling motor assembly 6 except for the mass body 54 are the same as those of the vibration canceling motor assembly 1 of FIG. 2, and thus a redundant description thereof will be omitted.

The mass body 54 may have a non-uniform density distribution in a radial direction perpendicular to the rotation axis of the shaft 21. For example, the mass body 54 may include an area where a density increases in the radial direction perpendicular to the rotation axis of the shaft 21. A center of mass CM₅₄ of the mass body 54 may be provided relatively far from the shaft 21 in the radial direction perpendicular to the rotation axis of the shaft 21, compared with a center of mass CM₅₁ of the mass body 51 of FIG. 3. Assuming that the mass body 51 of FIG. 3 has a uniform density, the center of mass CM₅₁ may be formed at an intersection between diagonal lines. However, even when it is assumed that the mass body 54 according to the present embodiment has the same mass, the same volume, and the same shape as the mass body 51 of FIG. 3, the center of mass CM₅₄ may be located farther from the shaft 21 in the radial direction perpendicular to the rotation axis of the shaft 21 than the center of mass CM₅₁. This mass body 54 may provide a high vibration attenuation effect by having a larger rotational inertia than the mass body 51 of FIG. 3.

FIG. 10 is a schematic cross-section of a vibration canceling motor assembly 7 according to another embodiment. Referring to FIG. 10, the vibration canceling motor assembly 7 includes a motor 11, a shaft 21, a driving force transmission member 31, an adhesion member 41, and a mass body 55. Components of the vibration canceling motor assembly 7 except for the mass body 55 are the same as those of the vibration canceling motor assembly 6 of FIG. 7, and thus a redundant description thereof will be omitted.

The mass body 55 may have a non-uniform density distribution in the radial direction perpendicular to the rotation axis of the shaft 21. For example, a density of the mass body 55 may increase in the radial direction perpendicular to the rotation axis of the shaft 21. For example, the mass body 55 may include a first mass body 55a, a second mass body 55b, and a third mass body 55c sequentially arranged in the radial direction perpendicular to the rotation axis of the shaft 21. A density of the first mass body 55a may be less than or equal to that of the second mass body 55b, and a density of the third mass body 55c may be less than or equal to that of the second mass body 55b.

Assuming that the mass body 51 of FIG. 3 has a uniform density, the center of mass CM₅₁ may be formed at an intersection between diagonal lines. However, even when it is assumed that the mass body 55 according to the present embodiment has the same mass, the same volume, and the same shape as the mass body 51 of FIG. 3, a center of mass CM₅₅ of the mass body 55 may be located farther from the shaft 21 in the radial direction perpendicular to the rotation axis of the shaft 21 than the center of mass CM₅₁. This mass body 55 may provide a high vibration attenuation effect by having a larger rotational inertia than the mass body 51 of FIG. 3.

Exemplary embodiments have been explained above and shown in the attached drawings in order to promote understanding of the present disclosure. It should be understood that the exemplary embodiments described therein should be considered in a descriptive sense only and not for purposes of limitation. Descriptions of features or aspects within each embodiment should typically be considered as available for other similar features or aspects in other embodiments, because various other modifications may be made by one of ordinary skill in the art to which the present disclosure pertains.

## Claims

1. A vibration canceling motor assembly comprising:
a motor;
a shaft that is rotated about a rotation axis of the shaft by the motor;
a driving force transmission member including a support area coupled to the shaft to be fixed to the shaft and a drive transmission area that transmits a driving force to outside;
a mass body arranged in at least a portion of the support area; and
an adhesion member interposed between the mass body and the driving force transmission member.

2. The vibration canceling motor assembly of claim 1, wherein the driving force transmission member has a hollow shape such that the shaft is inserted into and fixed to the driving force transmission member.

3. The vibration canceling motor assembly of claim 1 or 2, wherein
the shaft comprises a shaft groove formed on at least a portion of an outer circumferential surface of the shaft, and
the driving force transmission member comprises a protrusion inserted into the shaft groove to couple the shaft to the driving force transmission member.

4. The vibration canceling motor assembly of claim 3, wherein the shaft groove is provided on a leading end of the shaft.

5. The vibration canceling motor assembly of any of the preceding claims, further comprising a coupling member configured to couple the adhesion member to the mass body.

6. The vibration canceling motor assembly of claim 5, wherein the coupling member is further configured to couple the adhesion member to the driving force transmission member.

7. The vibration canceling motor assembly of any of the preceding claims, wherein a density distribution of the mass body is non-uniform in a radial direction perpendicular to the rotation axis of the shaft.

8. The vibration canceling motor assembly of claim 7, wherein a density of the mass body increases in the radial direction perpendicular to the rotation axis of the shaft.

9. The vibration canceling motor assembly of any of the preceding claims, wherein the driving force transmission member is a bending gear or a pulley gear.

10. The vibration canceling motor assembly of any of the preceding claims, wherein the adhesion member includes an elastic material having an elasticity.

11. The vibration canceling motor assembly of any of the preceding claims, wherein the mass body includes a metal material.

12. The vibration canceling motor assembly of any of the preceding claims, wherein a natural frequency of the mass body is substantially the same as a natural frequency of the motor.

13. The vibration canceling motor assembly of any of the preceding claims, wherein a natural frequency of the adhesion member is substantially the same as a natural frequency of the motor.

14. An ultrasound probe comprising the vibration canceling motor assembly of any of the preceding claims.

15. The ultrasound probe of claim 14, further comprising:
an ultrasound module including a transducer or a piezoelectric element and configured to generate ultrasound waves; and
a drive transmitter configured to transmit a drive from the vibration canceling motor assembly to the ultrasound module.
